# FASCICULE DE BREVET EUROPEEN

(11) **EP 0 757 904 B1**
(45) Date de publication et mention de la délivrance du brevet: **12.11.2003**
(21) Numéro de dépôt: 96401572.1
(22) Date de dépôt: 16.07.1996
(51) Int. Cl.: A61F 2/06

(54) **Prothèse implantable dans un conduit humain ou animal, telle qu'un élargisseur de paroi, ou une prothèse pour anévrisme**
Implantierbare Prothese für ein menschliches oder tierisches Gefäss, wie z.B. Stent oder Aneurismaprothese
Implantable prosthesis for a human or animal vessel such as stent or aneurysm prosthesis

(30) Priorité: 03.08.1995 FR 9509473
(43) Date de publication de la demande: 12.02.1997
(73) Titulaire: B. BRAUN CELSA, 86360 Chasseneuil du Poitou (FR)
(72) Inventeur: Nadal, Guy, 86000 Poitiers (FR); Chevillon, Gérard, 92120 Montrouge (FR); Cottenceau, Jean-Philippe, 92160 Antony (FR)
(74) Mandataire: Lerner, François

(56) Documents cités:
- EP-A- 0 421 729
- EP-A- 0 565 251
- EP-A- 0 657 147
- EP-A- 0 686 379
- WO-A-92/06734
- WO-A-95/08966
- US-A- 4 994 071

## Description

L'invention se rapporte au domaine des prothèses tubulaires implantables dans un conduit anatomique d'un corps humain ou animal, pour assurer (c'est-à-dire pour maintenir, ou rétablir éventuellement) un passage dans ledit conduit.

Parmi les prothèses de ce type, l'invention concerne en particulier les prothèses vasculaires, et, parmi ces dernières, tout particulièrement les élargisseurs ou étais de parois (également appelés "stents") ou encore, les prothèses pour anévrisme pourvues non seulement d'éléments structuraux comme les "stents", mais également d'une gaine d'habillage extérieur destinée à canaliser le flux sanguin et, en règle générale, de moyens d'ancrage à la paroi du vaisseau, tels que des crochets portés par les éléments structuraux précités.

Des exemples de telles prothèses existent dans US 4 994 071, US 4 856 516, EP 335 341, EP 117 330, EP 565 251 ou encore US 5 387 235 et EP 508 473.

Dans EP-A-0 565 251 ou WO-A-95/08966 ou EP-A-0 686 379, il est déjà connu une prothèse propre à être implantée dans un conduit humain ou animal pour assurer un passage dans le conduit, la prothèse présentant une surface tubulaire ayant un axe et comprenant une pluralité de filaments, chaque filament définissant une partie seulement de ladite surface tubulaire, ces filaments présentant chacun plusieurs ondulations successives, lesquelles sont enroulées pour définir la surface tubulaire, par tronçons de tube(s).

Toutefois, les prothèses existantes peuvent être considérées comme n'offrant pas nécessairement les conditions optimales de fiabilité et de compromis entre la nécessaire souplesse de la prothèse (compte tenu de son emplacement d'implantation) et sa rigidité, compte tenu de son action dans le conduit.

Dans ces conditions, l'invention a pour objet de proposer une prothèse particulièrement fiable d'utilisation, aisée à réaliser et à implanter, tout en offrant la possibilité d'être utilisée en tant que "stent" simple ou bifurqué, ou encore de servir d'élément structurel pour une prothèse pour anévrisme simple ou bifurquée, voire être employée dans d'autres applications, pour d'autres conduits anatomiques.

Pour atteindre cet objectif, la prothèse de l'invention se caractérise en particulier en ce que les filaments passent d'un tronçon de tube(s) à un autre.

Ainsi, on pourra réaliser avec un même fil plusieurs parties de tronçons de tube(s), diminuant ainsi d'autant les problèmes de jonction des filaments entre eux.

S'il s'agit d'une prothèse bifurquée, chaque filament concerné par l'une des branches définira par ailleurs avantageusement, pour cette branche, une partie seulement de la branche.

En d'autres termes, il est prévu dans l'invention la réalisation d'une prothèse médicale implantable dans un corps vivant comprenant une armature ayant un axe principal et comportant plusieurs niveaux de tronçons tubulaires (que l'on pourrait également qualifier d'anneaux) sensiblement coaxiaux formés individuellement par une structure à méandres, deux tronçons tubulaires étant reliés entre eux par au moins un segment de fil dont la longueur est adaptée pour qu'il passe d'un premier niveau (ou étage) de tronçon tubulaire à un second niveau de la structure.

Même si, dans le cadre de l'invention, les "tronçons de tube(s)" formés par les ondulations des filaments de la prothèse peuvent être agencés en hélice, il est malgré tout apparu préférable de conseiller que les tronçons tubulaires soient étagés suivant l'axe du tube considéré, chaque étage s'étendant radialement à l'axe, en étant défini par une succession d'ondulations de différents filaments disposées à la suite les unes des autres et enroulées en anneau fermé, un filament parmi la pluralité de filaments ne définissant donc qu'une partie d'un étage avant de passer à un autre étage.

De cette manière, lorsque la prothèse sera implantée et devra exercer une force radiale vers l'extérieur pour assurer le passage souhaité dans son conduit d'implantation, l'effort sera réparti radialement à l'axe considéré de la prothèse.

Pour favoriser l'implantation d'une prothèse par voie percutanée à l'intérieur d'un tube ou cathéter de très petit diamètre, de l'ordre de 2 à 5 mm, une autre caractéristique de l'invention prévoit que les ondulations des filaments seront avantageusement des zigzags présentant des sommets coudés reliés par des tronçons intermédiaires qui s'étendent transversalement à l'axe de tube considéré, en s'écartant angulairement les uns des autres, lorsque la prothèse présente un premier diamètre élargi, deux tronçons successifs de zigzags étant rapprochés les uns des autres pour s'étendre sensiblement parallèlement entre eux et audit axe de tube, alors que la prothèse présente un second diamètre inférieur au premier, pour permettre son implantation.

Lorsque, comme cela a été conseillé ci-avant, la prothèse de l'invention, et en particulier la prothèse bifurquée, comporte une série d'anneaux ondulés radiaux, la réalisation de la bifurcation pourra avantageusement s'obtenir, par étage, et à partir de l'étage où la branche principale se divise en ses deux branches secondaires, par fermeture sur elles-mêmes desdites ondulations des filaments en deux parties indépendantes disposées côte à côte, sans bien entendu que lesdites ondulations annulaires ainsi constituées soient liées entre elles, favorisant ainsi l'écartement naturel des deux branches.

Dans ce qui suit, on va maintenant décrire plus en détail différents modes de réalisation de l'invention, en référence aux dessins annexés, dans lesquels :
Les figures 1 et 2 représentent respectivement une première forme de réalisation d'une prothèse tubulaire simple, de type stent, en vue perspective et en vue développée à plat,
La figure 3 correspond au détail agrandi III de la figure 2,
Les figures 4 et 5 montrent une réalisation d'une prothèse bifurquée respectivement en vue perspective et en développé à plat,
La figure 6 montre en vue développée à plat une autre alternative de réalisation de la prothèse des figures 1 à 3,
Et les figures 7 et 8 se rapportent à la réalisation d'une prothèse tubulaire pour anévrisme.

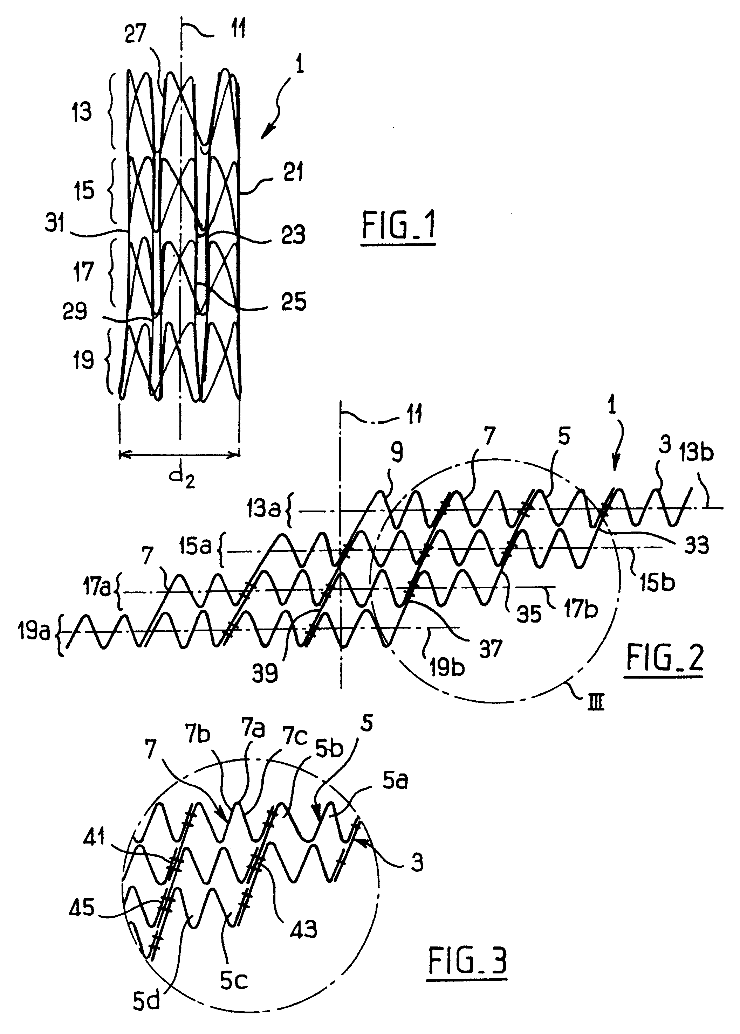

Sur la figure 1, tout d'abord, on voit donc illustré un élargisseur de vaisseau 1, couramment dénommé "stent", l'élément 1 pouvant également servir comme structure de base d'une prothèse pour anévrisme, voire d'une autre prothèse à implanter dans un autre conduit anatomique qu'un vaisseau, tel que par exemple la trachée ou l'oesophage.

Dans ce qui suit, nous appellerons d'une façon générale l'élément 1 : "stent", compte tenu de l'utilisation courante de ce terme.

Le stent 1 est basiquement constitué à partir de plusieurs filaments, en l'espèce au nombre de quatre, comme on peut le voir plus distinctement sur la figure 2 où lesdits filaments ont été repérés 3, 5, 7 et 9.11 peut s'agir en particulier de filaments métalliques, de quelques dixièmes de millimètre de diamètre, par exemple en alliage à base d'acier couramment utilisé pour nombre de prothèses vasculaires (filtre, stent, ...).

On remarquera que chacun des filaments 3, 5, 7, 9, présente des ondulations telles que repérées 5a, 5b, 5c, 5d, pour le fil 5 sur la figure 3.

Sur la figure 3, les ondulations sont conformée en zigzags avec, pour une zone du fil 7, des sommets courbés à angles assez aigus tels que 7a, raccordés entre eux alternativement dans un sens et dans l'autre par des tronçons intermédiaires tels que 7b, 7c, de préférence rectilignes.

La prothèse peut être réduite radialement à son axe longitudinal de tube 11, jusqu'à un premier diamètre d₁, pouvant être de l'ordre de 1,5 à 2,5 mm, de manière à pouvoir être introduite dans un fin tube introducteur permettant son implantation par voie percutanée, en particulier par la méthode connue dite de SELDINGER.

Si les filaments 3, 5, 7, 9, ont été choisis en métal de qualité ressort, la prothèse 1 pourra être de type radialement "auto-expansible", c'est-à-dire occuper son diamètre réduit d₁ dans un état contraint, de telle manière que si on annule cette contrainte radiale, la prothèse s'expansera radialement d'elle-même jusqu'à parvenir dans la position de la figure 1 (diamètre d₂) où les tronçons intermédiaires des filaments sont écartés angulairement les uns des autres par rapport à l'axe 11, alors que lorsque la prothèse présente son diamètre réduit d₁, lesdits filaments sont sensiblement plaqués les uns contre les autres, sensiblement parallèlement à cet axe 11, ainsi que cela a été déjà décrit dans EP-A-117 330.

Sur les illustrations, on a privilégié une disposition étagée des ondulations, de telle manière que, par exemple dans le cas d'une prothèse à quatre étages, les ondulations en zigzags de chaque étage définissent autant d'anneaux ou surfaces annulaires 13, 15, 17, 19, que d'étages 13a, 15a, 17a, 19a, ces anneaux s'étendant chacun suivant un axe, respectivement 13b, 15b, 17b, 19b, sensiblement perpendiculaire à l'axe longitudinal 11. Pour assurer la cohésion structurelle de la prothèse, certains au moins (en l'espèce chacun) des filaments passent d'un étage au suivant, en définissant ainsi des sortes de "stabilisateurs longitudinaux" 23, 25, 27, 29, 31, constitués par l'association, avec fixation entre eux par étage, des tronçons de jonction entre étages, tels que 33, 35, 37, 39.

Sur les figures, on a tiré parti de la forme en zigzags pour, à chaque étage, prolonger axialement l'un des segments intermédiaires de ces zigzags jusqu'à venir former le début de quelques ondulations de l'anneau de l'étage suivant, et ainsi de suite. Ainsi, chaque filament constitutif de la prothèse ne forme, à chaque étage, qu'une partie de la structure ondulée de l'anneau correspondant. Plus précisément, dans les réalisations illustrées, chaque structure annulaire, telle que 13, 15, 17, 19 sur la figure 1, est constituée par quatre tronçons ondulés appartenant aux quatre filaments constitutifs de la prothèse, chaque filament présentant à chaque étage quatre ondulations alternées, avant de passer à un autre étage pour constituer une autre structure annulaire radiale.

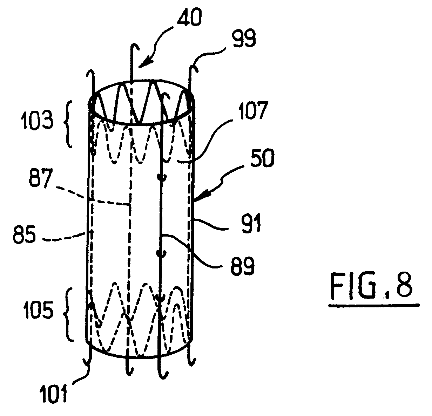

Dans la mesure où les illustrations des figures 1 à 6 se rapportent à des stents, les structures annulaires en question ont été étagées de manière qu'elles soient disposées de façon adjacente les unes à la suite des autres le long de l'axe 11 (à la différence des réalisations des figures 8 et suivantes, où une distance d de plusieurs centimètres sépare, suivant le même axe 11, deux structures ondulées annulaires radiales consécutives.

Pour réaliser la prothèse qui vient d'être décrite, on referme sur eux-mêmes chaque étage d'ondulations 13a, ..., 19a, suivant son axe radial respectif 13b, ..., 19b, en fixant entre elles, par exemple par quelques points de soudure, les extrémités des filaments.

Alors que sur la figure 2, chaque filament s'étend, étage après étage, d'une extrémité proximale à l'autre de la prothèse, la variante de la figure 3 montre que chaque filament peut être interrompu d'endroits en endroits, sur sa longueur, un même filament pouvant par exemple être réalisé en trois segments successifs soudés bout à bout, tel qu'aux emplacements repéré 41, 43 ou 45 sur la figure 3 (les petites barrettes transversales représentant des zones de soudage). Suivant les étages, on pourrait même avoir un nombre différent de filaments.

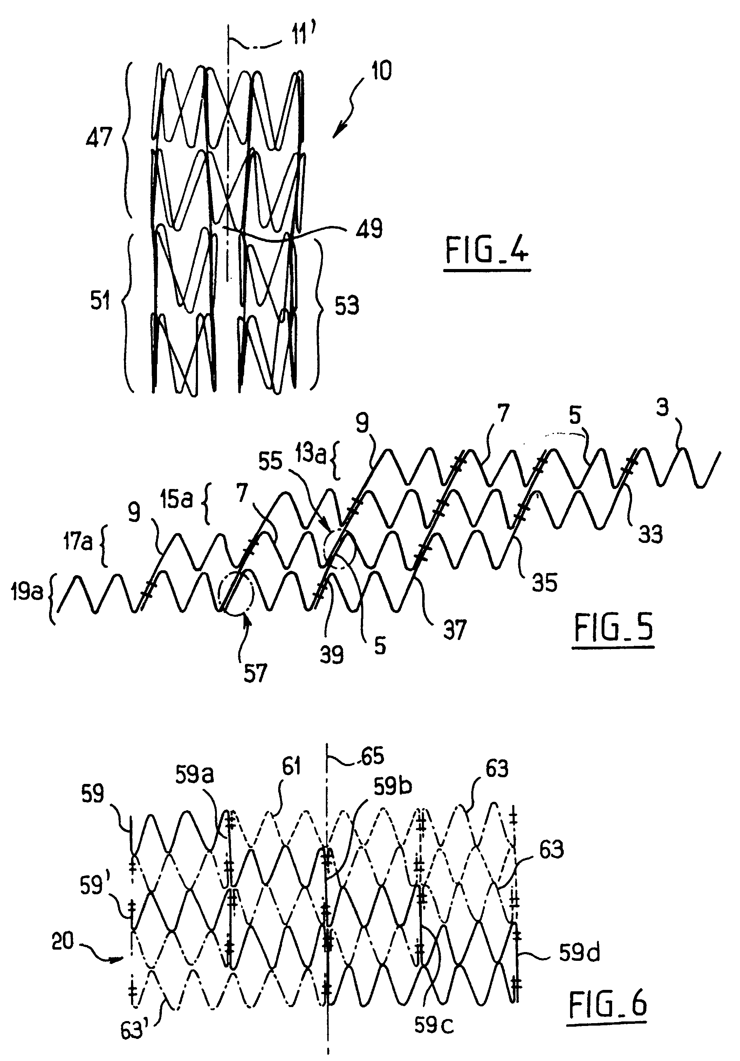

Sur la figure 4, on a illustré un stent bifurqué 10 comprenant une première branche ou tronc principal 47, par exemple jusqu'à environ la moitié, voire les deux tiers, de la longueur de la prothèse, ce tronc principal se divisant à partir d'une zone d'embranchement 49 en deux branches tubulaires secondaires (51, 53) prévues pour pouvoir être écartées l'une de l'autre angulairement par rapport à l'axe central 11'.

En étudiant la figure 5 et en la comparant à la figure 2, on notera que les deux prothèses 1 et 10 peuvent être réalisées de manière tout à fait identique, (y compris dans la variante de réalisation de la figure 3), sauf aux endroits entourés et fléchés 55, 57 sur la figure 5 où deux filaments adjacents placés côte à côte d'un même étage (en l'espèce, les filaments 5, 7 et 7, 9, respectivement pour les étages 17a et 19a) ne sont pas liés ensemble et demeurent donc disjoints. Sur les branches 51, 53, on notera en outre que le nombre d'ondulations par étage est inférieur à celui de la branche principale 47.

Sur la figure 6, les différents filaments de la prothèse 20 illustrée ont été repérés, pour les filaments 59 et 59', en trait continu, pour le filament 61, en tirets, et pour les filaments 63, 63', en traits mixtes.

Ce qui différencie (outre sa manière de le réaliser), le stent 20 des prothèses 1 et 10, est que les tronçons de changement d'étages sont ici dirigés parallèlement à l'axe longitudinal 65 et non pas en biais.

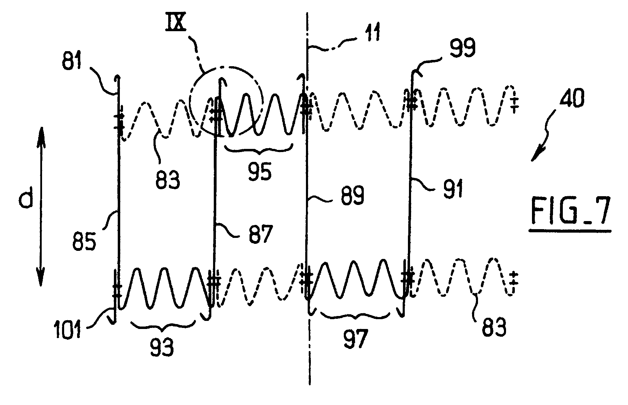

Sur la figure 7, c'est l'armature d'une prothèse pour anévrisme qui a été figurée. L'élément 40 en question est ainsi constitué de deux types de fins filaments métalliques 81, 83. En l'espèce, cinq filaments 83 identiques (ou éventuellement plus ou moins longs) sont utilisés, trois pour la portion supérieure, et deux pour la portion inférieure, de manière à définir respectivement une zone annulaire ondulée supérieure et une autre zone identique inférieure, toutes deux radiales à l'axe de tube 11, ces deux zones étant bien entendu séparées par la distance d. Entre ces deux zones, s'étendent des parties rectilignes formant les stabilisateurs longitudinaux 85, 87, 89, 91. Ceux-ci s'étendent parallèlement à l'axe central de tube 11 et appartiennent au filament 81 qui présente, alternativement aux étages supérieur et inférieur de la prothèse les tronçons en zigzags, 93, 95 et 97, auxquels sont bien entendu fixés, par soudage, à l'étage approprié, respectivement les trois tronçons supérieurs de filaments 83 et les deux tronçons inférieurs.

Pour une fixation de la structure 40 dans son conduit d'implantation (qui peut en particulier être une artère), ladite structure est en outre pourvue de crochets d'ancrage 99, 101, qui peuvent être soit constitués par les extrémités opposées recourbées en crochet du filament 81, soit par des petites barrettes également recourbées en crochet à leurs extrémités, 101, fixées par exemple par soudage, sensiblement dans le prolongement axial des barrettes 85, 87, 89, 91, pour retenir axialement, dans un sens et/ou dans l'autre la structure.

Sur la figure 8, on a représenté en vue schématique d'ensemble une prothèse pour anévrisme 50, avec son armature 40 de la figure 8 et notamment ses quatre stabilisateurs axiaux 85, 87, 89, 91, ses deux structures-ressorts en zigzags supérieur et inférieur, respectivement 103 et 105, ses crochets 99, 101, ainsi bien entendu que sa gaine d'habillage 107 pour la canalisation du flux sanguin à travers elle. Pour tout renseignement concernant la gaine 107, et sa liaison avec son armature de soutien, on se reportera à FR-A-2 693 366.

Dans ce qui précède, à la place de filaments de "qualité ressort", on pourrait employer des filaments à mémoire de forme (par exemple thermique ; "Nitinol", marque déposée) ou des filaments expansibles par ballonnement.

## Revendications

1. Prothèse (1, 20, 30) propre à être implantée dans un conduit humain ou animal pour assurer un passage dans le conduit, la prothèse présentant une surface tubulaire ayant un axe et comprenant une pluralité de filaments (3, 5, 7, 9), chaque filament définissant une partie seulement de la surface tubulaire, ces filaments présentant chacun plusieurs ondulations successives, lesquelles sont enroulées pour définir la surface tubulaire, par tronçons de tube(s) (13a, 15a, 17a, 19a), **caractérisée en ce que** les filaments passent d'un tronçon de tube(s) à un autre (35, 37, 39).

2. Prothèse selon la revendication 1, **caractérisée en ce qu'**il s'agit d'une prothèse bifurquée (10) qui présente une branche tubulaire principale se divisant en deux branches tubulaires secondaires ayant chacune un axe, les ondulations des filaments étant enroulées pour définir lesdites branches tubulaires, respectivement, par tronçons de tube(s), chaque filament définissant, pour la (les) branches concernée(s), une partie seulement de cette (ces) branche(s).

3. Prothèse selon la revendication 1 ou la revendication 2, **caractérisée en ce que** les tronçons tubulaires sont étagés suivant l'axe du tube considéré, chaque étage (13a, 15a, ...) s'étendant radialement à l'axe, en étant défini par une succession d'ondulations de différents filaments disposées à la suite les unes des autres et enroulées en anneau fermé (13, 15, 17, 19), un filament parmi la pluralité de filaments ne définissant donc qu'une partie d'un étage ou tronçon de tube (13a, 15a, ...) avant de passer à un autre étage.

4. Prothèse selon l'une quelconque des revendications précédentes, **caractérisée en ce que** lesdites ondulations sont des zigzags et présentent ainsi des sommets coudés (7a) reliés par des tronçons intermédiaires (7b, 7c) qui s'étendent transversalement à l'axe considéré, en s'écartant angulairement les uns des autres, lorsque la prothèse présente un premier diamètre élargi, deux tronçons intermédiaires successifs de zigzags étant rapprochés les uns des autres pour s'étendre sensiblement parallèlement entre eux et audit axe, lorsque la prothèse présente un second diamètre inférieur au premier, pour permettre son implantation.

5. Prothèse selon les revendications 1 ou 2, et 4, **caractérisée en ce que** certains au moins des filaments passent d'un tronçon de tube (13, 15, 17, 19) au tronçon de tube suivant par l'intermédiaire d'un tronçon du filament (35, 37, 39) correspondant s'étendant transversalement par rapport à l'axe considéré.

6. Prothèse selon l'une quelconque des revendications précédentes, **caractérisée en ce que** le nombre d'ondulations de chaque filament, ou de filaments, par tronçon de tube(s) est différent pour certains tronçons par rapport à d'autres.

7. Prothèse selon l'une des revendications 2 ou 3, **caractérisée en ce qu'**à partir de l'étage où la branche principale (47) se divise en branches secondaires (51, 53), les ondulations des filaments se ferment sur elles-mêmes par étage en deux parties indépendantes disposées côte à côte, pour définir respectivement le tronçon de tube correspondant de chaque branche.

## Claims

1. Prosthesis (1, 20, 30) for implantation in a human or animal duct to ensure a passageway in said duct, said prosthesis having a tubular surface and a tube axis and comprising a plurality of filaments (3, 5, 7, 9), each filament defining only a portion of said tubular surface, said filaments each having several successive corrugated portions, which are rolled up for defining said tubular surface, by tubular portions (13a, 15a, 17a, 19a), **characterized in that** said filaments go from a tubular portion to another one (35, 37, 39).

2. Prosthesis according to claim 1, **characterized in that** it constitutes a forked prosthesis (10) comprising a tubular main branch dividing into two secondary tubular branches each having an axis, said corrugated portions being rolled up for defining said tubular branches, respectively, by tubular portions, each filament defining, for the related branch(es), a portion only of said branch(es).

3. Prosthesis according to claim 1 or claim 2, **characterized in that** said tubular portions are stepped along the related tube axis, each step (13a, 15a,...) extending radially from said axis and being defined by a sequence of corrugated portions of different filaments sequentially disposed and rolled into a closed ring (13, 15, 17, 19), a filament from said plurality of filaments then defining only a portion of a step or tubular portion (13a, 15a,...) before going to an other step.

4. Prosthesis according to anyone of preceding claims, **characterized in that** said corrugated portions comprise zig-zags and present V-shaped apices (7a) connected by intermediate portions (7b, 7c) extending in skewed relation with said axis, and being angularly spaced relatively to each other when said prosthesis has a first enlarged diameter, two successive intermediate portions of zig-zags being adjacent to each other for extending generally in parallel to each other and to said axis when said prosthesis has a second diameter smaller than first diameter for allowing implantation.

5. Prosthesis according to claims 1 or 2, and 4, **characterized in that** at least some of said filaments go from a tubular portion (13, 15, 17, 19) to the next tubular portion through a corresponding filament section (35, 37, 39) extending in skewed relation with said axis.

6. Prosthesis according to anyone of preceding claims, **characterized in that** the number of corrugated portions of each filament or of filaments, by tubular portion, is different for some portions from that of other portions.

7. Prosthesis according to any of claims 2 or 3, **characterized in that**, from the step where said main branch (47) divides into secondary branches (51, 53), said corrugated portions close on themselves by step into two independent portions disposed side-by-side, for defining said respective tubular portion corresponding to each branch.

## Patentansprüche

1. Prothese (1, 20, 30) zum Implantieren in einen menschlichen oder tierischen Gang, um einen Durchgang in diesem Gang sicherzustellen, wobei die Prothese eine röhrenförmige Oberfläche mit einer Achse und eine Vielzahl von Filamenten (3, 5, 7, 9) aufweist, jedes Filament nur einen Teil der röhrenförmigen Oberfläche bestimmt und diese Filamente jedes mehrere aufeinanderfolgende Wellenlinien aufweist, die eingerollt sind, um die röhrenförmige Oberfläche in Abschnitten des Rohres (von Rohren) (13,a, 15a, 17a, 19a) zu bilden, **dadurch gekennzeichnet, daß** die Filamente von einem Abschnitt des Rohres (der Rohre) zu einem anderen (35, 37, 39) gehen.

2. Prothese nach Anspruch 1, **dadurch gekennzeichnet, daß** es sich um eine gegabelte Prothese (10) handelt, die einen röhrenförmigen Hauptzweig aufweist, der sich in zwei röhrenförmige Nebenzweige teilt, von denen jeder eine Achse hat, wobei die Wellenlinien der Filamente aufgerollt sind, um die röhrenförmigen Zweige jeweils in Abschnitten des Rohres (der Rohre) zu bestimmen und jedes Filament für den betreffenden Zweig (die betreffenden Zweige) nur einen Teil dieses Zweiges (dieser Zweige) bestimmt.

3. Prothese nach Anspruch 1 oder Anspruch 2, **dadurch gekennzeichnet, daß** die röhrenförmigen Abschnitte entlang der Achse des betreffenden Rohres abgestuft sind, wobei sich jede Stufe (13a, 15a, ...) radial zu der Achse erstreckt und durch eine Folge von Wellenlinien verschiedner Filamente bestimmt ist, die nacheinander angeordnet und zu einem geschlossenen Ring (13, 15, 17, 19) aufgerollt sind, wobei ein Filament unter der Vielzahl der Filamente also nur einen Teil einer Stufe oder Rohrabschnittes (13a, 15a, ...) bestimmt, bevor es zu einer anderen Stufe geht.

4. Prothese nach einem der vorhergehenden Ansprüche, **dadurch gekennzeichnet, daß** die Wellenlinien Zickzackform und somit gebogene Scheitel (7a) haben, die durch Zwischenabschnitte (7b, 7c), welche sich quer zu der betreffenden Achse erstrecken, verbunden sind, wobei sie sich winkelig zueinander im Abstand befinden, wenn die vorliegende Prothese einen ersten vergrößerten Durchmesser hat, und zwei aufeinanderfolgende Zwischenabschnitte in Zickzackform einander angenähert sind, um sich im wesentlichen parallel zueinander und zu der Achse zu erstrecken, wenn die Prothese einen zweiten Durchmesser aufweist, der kleiner als der erste ist, um ihre Implantation zu erlauben.

5. Prothese nach den Ansprüchen 1 oder 2 und 4, **dadurch gekennzeichnet, daß** wenigstens einige der Filamente von einem Rohrabschnitt (13, 15, 17, 19) zu dem folgenden Rohrabschnitt mittels eines entsprechenden Abschnittes des Filamentes (35, 37, 39), der sich quer zu der betreffenden Achse erstreckt, geht.

6. Prothese nach einem der vorhergehenden Ansprüche, **dadurch gekennzeichnet, daß** die Anzahl der Wellenlinien jedes Filamentes oder der Filamente in dem Abschnitt des Rohres (der Rohre) für bestimmte Abschnitte im Vergleich zu anderen unterschiedlich ist.

7. Prothese nach einem der Ansprüche 2 oder 3, **dadurch gekennzeichnet, daß** ausgehend von der Stufe, wo sich der Hauptzweig (47) in Nebenzweige (51, 53) teilt, sich die Wellenlinien der Filamente auf sich selbst stufig in zwei unabhängigen Teilen schließen, die Seite an Seite angeordnet sind, um jeweils den entsprechenden Abschnitt des Rohres jedes Zweiges zu bestimmen.
